(19) Europäisches Patentamt
European Patent Office
Office européen des brevets

(11) Publication number: 0 225 746 A2

(12) **EUROPEAN PATENT APPLICATION**

(21) Application number: 86308905.8

(22) Date of filing: 14.11.86

(51) Int. Cl.⁴: C 07 K 7/06, C 07 K 7/20, A 61 K 37/02

(30) Priority: 14.11.85 US 798239
27.06.86 US 879338

(43) Date of publication of application: 16.06.87 Bulletin 87/25

(84) Designated Contracting States: AT BE CH DE ES FR GB GR IT LI LU NL SE

(71) Applicant: The Administrators of The Tulane Educational Fund, 1430 Tulane Avenue, New Orleans Louisiana 70112 (US)

(72) Inventor: Coy, David H., 4319 Perrier Street, New Orleans§Louisiana 70115 (US)
Inventor: Moreau, Jacques-Pierre, 159 Westboro Road, Upton§Massachusetts 01568 (US)

(74) Representative: Sheard, Andrew Gregory et al, Kilburn & Strode 30, John Street, London WC1N 2DD (GB)

(54) **Therapeutic decapeptides.**

(57) A decapeptide of the formula:

N-Ac-$A^1$-$A^2$-$A^3$-Ser-$A^4$-$A^5$-$A^6$-$A^7$-$A^8$-$A^9$, wherein each $A^1$, $A^2$, and $A^3$, independently, is D-β-Nal, D-p-X-Phe (where X is halogen, H, $NH_2$, $NO_2$, OH, or $C_{1-3}$ alkyl), D-Trp, D-benzothienyl (2)-Ala, or D-benzothienyl (1)-Ala; $A^4$ is p-X-Phe (where X is halogen, H, $NH_2$, $NO_2$, or $C_{1-3}$ alkyl), Tyr, Lys, Arg, Leu, Trp, or Nal; $A^5$ is D-Lys, D-Tyr, D-Arg, D-Phe, D-β-Nal, D-Trp, D-homo-Arg, D-diethyl-homo-Arg, D-p-X-Phe (where X is halogen, H, $NH_2$, $NO_2$, or $C_{1-3}$ alkyl), or D-Lys-ε-NH-R (where R is H, a branched or straight chain $C_1$-$C_{10}$ akyl group, or an aryl group); $A_6$ is Leu, β-Nal, p-X-Phe (where X is halogen, H, $NH_2$, $NO_2$, OH, $C_2F_5$, $C_{1-3}$ alkyl), or Trp; $A^7$ is Arg, Lys, or Lys-ε-NH-R (where R is H, a branched or straight chain $C_1$-$C_6$ alkyl group, or an aryl group); $A_8$ is Pro; and $A^9$ is D-Ala, D-Ala-$NH_2$, Ala-$NH_2$, aminoisobutyric acid amide, or Gly-$NH_2$; provided that at least one of $A^2$ or $A^3$ must be D-Phe or D-Tyr, and provided further that when $A^4$ is Lys or Arg, $A^5$ must not be D-Arg, D-Lys, D-homo-Arg, D-diethyl-homo-Arg, or D-Lys-ε-NH-R, or a pharmaceutically acceptable salt thereof, can be used to inhibit the release of sex hormones in the treatment or prophylaxis of various sex hormone-released conditions. They may also be used as female contraceptives.

EP 0 225 746 A2

# THERAPEUTIC DECAPEPTIDES

This invention relates to therapeutic peptides.

A number of luteinizing hormone releasing hormone (LH-RH) analogs have been described which inhibit the release of LH-RH, a peptide hormone having the formula pGlu-His-Trp-Ser-Tyr-Gly-Leu-Arg-Pro-Gly-$NH_2$. For example, Coy et al. U.S. Patent No. 4,431,635, hereby incorporated by reference, describes LH-RH analogs having the general formula

X-$R^1$-$R^2$-$R^3$-Ser-Tyr-$R^4$-Leu-Arg-Pro-$R^5$-$NH_2$, in which X can be Ac; $R^1$ and $R^4$, independently, can be D-Trp or D-p-X-Phe, where X is a halogen or methyl group; $R^2$ can be D-p-X-Phe; $R^3$ can be D-Trp; and $R^5$ can be Gly or D-Ala.

In general, the present invention features a decapeptide of the formula:

N-Ac-$A^1$-$A^2$-$A^3$-Ser-$A^4$-$A^5$-$A^6$-$A^7$-$A^8$-$A^9$, wherein each $A^1$, $A^2$, and $A^3$, independently, is D- β -Nal, D-p-X-Phe (where X is halogen, H, $NH_2$, $NO_2$, OH, or $C_{1-3}$ alkyl), D-Trp, D-benzothienyl (2)-Ala, or D-benzothienyl (1)-Ala; $A^4$ is p-X-Phe (where X is halogen, H, $NH^2$, $NO_2$, or $C_{1-3}$ alkyl), Tyr, Lys, Arg, Leu, Trp, or Nal; $A^5$ is D-Lys, D-Tyr, D-Arg, D-Phe, D- β -Nal, D-Trp, D-homo-Arg, D-diethyl-homo-Arg, D-p-X-Phe (where X is halogen, H, $NH_2$,

$NO_2$, or $C_{1-3}$ alkyl) or D-Lys-ε -NH-R (where R is H, a branched or straight chain lower ($C_1$-$C_{10}$) alkyl group, e.g., methyl, isopropyl, heptyl or butyl, or an aryl group, e.g., benzyl, p-Cl-benzyl, or $CH_2$-naphthyl); $A_6$ is Leu, β-Nal, p-X-Phe (where X is halogen, H, $NH_2$, $NO_2$, OH, $C_2F_5$, or $C_{1-3}$ alkyl), or Trp; $A^7$ is Arg, Lys, or Lys-ε -NH-R (where R is H, a branched or straight chain lower ($C_1$-$C_6$) alkyl group, e.g., methyl, isopropyl, heptyl or butyl, or an aryl group, e.g., benzyl, p-Cl-benzyl, or $CH_2$-naphthyl); $A^8$ is Pro; and $A^9$ is D-Ala, D-Ala-$NH_2$, Ala-$NH_2$, aminoisobutyric acid amide, or Gly-$NH_2$; provided that at least one of $A^2$ or $A^3$ must be D-Phe or D-Tyr, and provided further that when $A^4$ is Lys or Arg, $A^5$ must not be D-Arg, D-Lys, D-homo-Arg, D-diethyl-homo-Arg, or D-Lys-ε -NH-R, or a pharmaceutically acceptable salt thereof. (β-Nal refers to β-napththylalanine; where no L- or D- designation is given herein, the L-isomer is intended.)

In one preferred decapeptide, $A^1$ is D-β-Nal, $A^2$ is D-p-Cl-Phe, $A^3$ is D-Phe, $A^4$ is Tyr, $A^5$ is D-Arg, $A^6$ is Phe, $A^7$ is Arg, $A^8$ is Pro, and $A^9$ is D-Ala.

In another preferred decapeptide, $A^1$ is D-β-Nal, $A^2$ is D-Phe, $A^3$ is D-Phe, $A^4$ is Tyr, $A^5$ is D-Arg, $A^6$ is Phe, $A^7$ is Arg, $A^8$ is Pro, and $A^9$ is D-Ala.

In another preferred decapeptide, $A^1$ is D-β-Nal, $A^2$ is D-Phe, $A^3$ is D-Phe, $A^4$ is Tyr, $A^5$ is D-Lys (isopropyl), $A^6$ is Phe, $A^7$ is Lys (isopropyl), $A^8$ is Pro, and $A^9$ is Ala-$NH_2$.

In another preferred decapeptide, $A^1$ is D-β-Nal, $A^2$ is D-p-Cl-Phe, $A^3$ is D-Tyr, $A^4$ is Phe, $^5$ is D-Arg, $A^6$ is Leu, $A^7$ is Arg, $A^8$ is Pro, and $A^9$ is D-Ala.

In another preferred decapeptide, $A^1$ is D-β-Nal, $A^2$ is D-Phe, $A^3$ is D-Phe, $A^4$ is Phe, $A^5$ is D-Arg, $A^6$ is Leu, $A^7$ is Arg, $A^8$ is Pro, and $A^9$ is D-Ala.

In another preferred decapeptide, $A^1$ is D-β-Nal, $A^2$ is D-Phe, $A^3$ is D-Phe, $A^4$ is Tyr, $A^5$ is D-Lys (isopropyl), $A^6$ is Phe, $A^7$ is Arg, $A^8$ is Pro, and $A^9$ is D-Ala-$NH_2$.

In other preferred embodiments, a therapeutically effective amount of the therapeutic decapeptide and a pharmaceutically acceptable carrier substance, e.g., magnesium carbonate or lactose, together form a therapeutic composition for inhibiting the release of sex hormones, particularly LH, induced by LH-RH. This composition can be in the form of a pill, tablet, capsule, liquid, or sustained release tablet for oral administration; a liquid spray for nasal administration; or a liquid for intravenous, subcutaneous, parenteral, or intraperitoneal administration.

Another preferred form for administration is an injectible suspension of the peptide with a bioerodible, biocompatible polymer matrix capable of effecting sustained release of the peptide. Other suitable forms are peptide/polymer implants, transdermal patches, and compositions usable with iontophoretic techniques.

The decapeptides of the invention are active in inhibiting the LH-RH induced release of LH, and exhibit a long duration of activity, thus minimizing the amount and frequency of dosages. Furthermore, manufacture is

relatively simple and inexpensive. In addition, the peptides have the advantage of being able to be administered orally, a property owing at least in part to their high lipophilicity.

The peptides of the invention have D-Phe or D-Tyr at at least one of positions $A^2$ or $A^3$. D-Phe or D-Tyr at position $A^3$ have been found to be modifications of particular importance in terms of activity, while D-Phe at position $A^2$ provides further cost reduction, compared to D-p-X-Phe at $A^2$, without significant comparative loss of activity. D-Phe at $A^2$ and D-Tyr at $A^3$ also lessen the irritant effect of the decapeptide, as does the presence of D-Lys- ε -NH-R at position $A^5$ and Lys- ε -NH-R at position $A^7$ when R is an alkyl or aryl group. Presumably, this is due to an observed decrease in histamine-releasing activity. It is further believed that the presence of Arg at position $A^4$ and D-Tyr at position $A^5$, known as the Hodgen modification, also decreases histamine-releasing activity. p-X-Phe at position $A^6$ is also particularly advantageous in terms of activity.

Other features and advantages of the invention will be apparent from the following description of the preferred embodiments thereof, and from the claims.

We now describe the structure, synthesis, and use of preferred embodiments of the invention.

Structure

The decapeptides of the invention have the general formula recited above. They all have an acetyl group at the amino terminal end in addition to Ser at position 4. Substitution of non-natural substituents at positions other than $A^2$, $A^3$, and $A^6$ can be used to modify

the properties of the compound, and will not prevent the $A^2$, $A^3$, and/or $A^6$ substituents from providing their beneficial effects.

The decapeptides can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluenesulfonic, trifluoroacetic, or pamoic acid, as well as polymeric acids such as tannic acid or carboxymethyl cellulose, and salts with inorganic acids such as the hydrohalic acids, e.g., hydrochloric acid, sulphuric acid, or phosphoric acid.

Synthesis

Example 1

The synthesis of N-Ac-D- β -Nal-D-Phe-D-Phe-Ser-Tyr-D-Arg-Phe-Arg-Pro-D-Ala follows.

Other decapeptides of the invention can be prepared by making appropriate modifications of the following synthetic method.

The first step is the preparation of N-Acetyl-D- β -Nal- D-Phe-D-Phe-benzyl-Ser-Tyr-D-tosyl-Arg-Phe-tosyl-Arg-Pro-D-Ala-benzhydrylamine-resin, as follows.

Benzyhydrylamine-polystyrene resin (Bachem, Inc.) (1.00 g, 0.3 mmole) in the chloride ion form is placed in the reaction vessel of a Beckman 990B peptide synthesizer programmed to perform the following reaction cycle: (a) $CH_2Cl_2$; (b) 33% trifluoroacetic acid in $CH_2Cl_2$ (2 times for 1 and 25 min each); (c) $CH_2Cl_2$; (d) ethanol; (e) $CH_2Cl_2$; (f) triethylamine in $CHCl_3$; and (g) $CH_2Cl_2$.

The neutralized resin is stirred with alpha-t-butoxycarbonyl (Boc)-D-Ala and diisopropylcarbodiimide (1.5 mmole) in $CH_2Cl_2$ for 1 hour and the resulting

amino acid resin is then cycled through steps (a) to (g) in the above wash program. The following amino acids (1.5 mmole) are then coupled successively by the same procedure: Boc-Pro, Boc-Tosyl-Arg, Boc-Phe, Boc-Tosyl-D-Arg, Boc-Tyr, Boc-benzyl-Ser, Boc-D-Phe, and Boc-D-β-Nal.

After removal of the N-terminal Boc group, the peptide-benzyhydrylamine resin is neutralized and acetylated by treatment with 5% acetic acid in $CH_2Cl_2$. The completed resin is then washed with $CH_3OH$ and air dried.

From the above resin is prepared N-Ac-D-β-Nal-D-Phe-D-Phe-Ser-Tyr-D-Arg-Phe-Arg-Pro-D-Ala, as follows.

A mixture of the above decapeptide resin (1.85 g, 0.5 mmole) and a solution of 4 ml anisole, 100 mg dithiothreitol, and 36 ml hydrogen fluoride is stirred at 0°C for 45 minutes. Excess hydrogen fluoride is evaporated rapidly under a stream of dry nitrogen, after which the free peptide is precipitated and washed with ether.

The peptide is then dissolved in a minimum volume of 50% acetic acid and eluted on a column (2.5 X 100 mm) of Sephadex G-25. Fractions containing a major component, as determined by u.v. absorption and thin layer chromatography (tlc), are pooled and evaporated to a small volume *in vacuo*. This solution is applied to a column (2.5 X 50 cm) of octadecylsilane-silica (Whatman LRP-1, 15-20 um mesh size) which is eluted with a linear gradient of 15-50% acetonitrile in 20% acetic acid in water. Fractions are examined by tlc and analytical high performance liquid chromatography (hplc) and pooled

to give maximum purity. Repeated lyophilization of the solution from water gives 117 mg of the product as a white, fluffy powder.

This material is found to be homogeneous by hplc and tlc. Amino acid analysis of an acid hydrolysate confirms the composition of hte decapeptide.

N-Ac-D- β -Nal-D-p-Cl-Phe-D-Tyr-Ser-Phe-D-Arg-Leu-Arg-Pro-D-Ala was prepared according to the synthesis described above, substituting Boc-D-p-Cl-Phe for Boc-D-Phe at position $A^2$, Boc-D-Trp for Boc-D-Phe at position $A^3$, Boc-Phe for Boc-Tyr at position $A^4$, and Boc-Leu for Boc-Phe at $A^6$.

N-Ac-D- β -Nal-D-p-Cl-Phe-D-Tyr-Ser-Nhe-D-Arg-Leu-Arg-Pro-D-Ala was prepared according to the synthesis described above, substituting Boc-D-p-Cl-Phe for Boc-D-Phe at position $A^2$, Boc-D-Tyr for Boc-D-Phe at position $A^3$, Boc-Phe for Boc-Tyr at position $A^4$, and Boc-Leu for Boc-Phe at position $A^6$.

N-Ac-D- β -Nal-D-Phe-D-Phe-Ser-Phe-D-Arg-Leu-Arg-Pro-D-Ala was prepared according to the synthesis described above, substituting Boc-Phe for Boc-Tyr at $A^4$ and Boc-Leu for Boc-Phe at $A^6$.

Example 2

To synthesize peptides featuring D-Lys- ε -NH-R at position $A^5$ or Lys- ε -NH-R at position 7, where R is an alkyl or aryl group, a carbonyl-containing compound, e.g., acetone or formaldehyde, is reacted with a resin-bound polypeptide featuring a Lys or D-Lys subunit in the presence of sodium cyanoborohydride. The carbonyl-containing compound reacts with the free ε - $NH_2$ group on the side chain of the Lys or D-Lys subunit; reaction with acetone produces an ε - N-isopropyl moiety, whereas reaction with formaldehyde produces an ε -N-methyl moiety.

The synthesis of Ac-D- β -Nal-D-Phe-D-Phe-Ser-Tyr-D-Lys (isopropyl)-Phe-Lys (isopropyl)-Pro-Ala-$NH_2$ follows.

Ac-D- β -Nal-D-Phe-D-Phe-Ser (Bzl)-Tyr-D-Lys(FMOC)-Phe-D-Lys (FMOC)-Pro-D-Ala-benzhydrylamine resin (BzL=benzyl; FMOC=fluorenylmethyloxycarbonyl) was prepared by standard methods in a Beckman 990B automatic peptide synthesizer using 33% TFA (trifluoroacetic acid) for removal of the α -BOC protecting groups. The ε -FMOC protecting groups on the Lys residues are completely stable to these acidic conditions, and to subsequent neutralization steps with 10% triethylamine in chloroform. The resin was then treated with 50ml of a 50% solution of piperidine in DMF (dimethylformamide) for about 12h to remove the FMOC protecting groups from the Lys residues.

To react the free ε -amino groups of the Lys residues, the resin (0.25 mmole) was mixed with 5ml of acetone, and 1 mmole of sodium cyanoborohydride in DMF/1% acetic acid added. The resin mixture was then stirred until it was negative to ninhydrin reaction (about 3h); the negative ninhydrin reaction indicated that the free ε -amino groups had been converted to N-isopropyl amino groups.

The resin was then cleaved from the support by treatment with HF/anisole and purified under standard conditions to yield the desired polypeptide.

Ac-D-Nal-D-Phe-D-Phe-Ser-Tyr-D-Lys(isopropyl)-Phe-Arg-Pro-D-Ala-amide is prepared in analogous fashion using appropriate modifications of the above-described procedure.

Use

When administered to a mammal (e.g., orally, intravenously, parenterally, nasally, or by

suppository), the decapeptides are effective in inhibiting the release of LH induced by LH-RH.

The decapeptides of the invention can be used for the treatment of precocious puberty, hormone dependent tumours (e.g., malignant and benign prostatic, mammary, ovarian and testicular tumors), hirsutism, acne, amenorrhea (e.g., secondary amenorrhea), endometriosis, and ovarian and mammary cystic diseases; the particular decapeptide described above is particularly effective in preventing the growth of mammary tumours. The decapeptides can also be used to regulate human menopausal gonadotropin luteinizing hormone (LH) and follicle-stimulating hormone (FSH) during perimenopausal and postmenopausal periods in women. The decapeptides can also be used as female contraceptives.

The decapeptides can be administered to a patient in a dosage of 10 mcg/kg/day to 1000 mcg/kg/day, preferably 25-250 mcg/kg/day.

Other embodiments are within the following claims.

CLAIMS FOR THE CONTRACTING STATES: BE, FR, DE, IT, LU, NL, SE, CH, LI and GB

1. A decapeptide of the formula:

N-Ac-$A^1$-$A^2$-$A^3$-Ser-$A^4$-$A^5$-$A^6$-$A^7$-$A^8$-$A^9$, wherein each $A^1$, $A^2$, and $A^3$, independently, is D- β -Nal, D-p-X-Phe (where X is halogen, H, $NH_2$, $NO_2$, OH, or $C_{1-3}$ alkyl), D-Trp, D-benzothienyl (2)-Ala, or D-benzothienyl (1)-Ala; $A^4$ is p-X-Phe (where X is halogen, H, $NH_2$, $NO_2$, or $C_{1-3}$ alkyl), Tyr, Lys, Arg, Leu, Trp, or Nal; $A^5$ is D-Lys, D-Tyr, D-Arg, D-Phe, β -Nal, D- β -Nal, D-Trp, D-homo-Arg, D-diethyl-homo-Arg, D-p-X-Phe (where X is halogen, H, $NH_2$, $NO_2$, or $C_{1-3}$ alkyl) or D-Lys- ε -NH-R (where R is H, a branched or straight chain $C_1$-$C_{10}$ alkyl group, or an aryl group); $A_6$ is Leu, β -Nal, p-X-Phe (where X is halogen, H, $NH_2$, $NO_2$, OH, $C_2F_5$, or $C_{1-3}$ alkyl), or Trp; $A^7$ is Arg, Lys, or Lys- ε -NH-R (where R is H, a branched or straight chain $C_1$-$C_{10}$ alkyl group, or an aryl group); $A_8$ is Pro; and $A^9$ is D-Ala, D-Ala-$NH_2$, Ala-$NH_2$, aminoisobutyric acid amide, or Gly-$NH_2$; provided that at least one of $A^2$ or $A^3$ must be D-Phe or D-Tyr, and provided further that when $A^4$ is Lys or Arg, $A^5$ must not be D-Lys, D-Arg, D-homo-Arg, D-diethyl-homo-Arg, or D-Lys- ε -NH-R, or a pharmaceutically acceptable salt thereof.

2. A decapeptide as claimed in claim 1 wherein $A^3$ is D-Phe.

3. A decapeptide as claimed in claim 1 or 2 wherein $A^2$ is D-Phe.

4. A decapeptide as claimed in claim 1, 2 or 3, wherein $A^6$ is p-X-Phe.

5. A decapeptide as claimed in claim 1 wherein $A^1$ is D-$\beta$-Nal, $A^2$ is D-p-Cl-Phe, $A^3$ is D-Phe, $A^4$ is Tyr, $A^5$ is D-Arg, $A^6$ is Phe, $A^7$ is Arg, $A^8$ is Pro, and $A^9$ is D-Ala; or

wherein $A^1$ is D-$\beta$-Nal, $A^2$ is D-Phe, $A^3$ is D-Phe, $A^4$ is Tyr, $A^5$ is D-Arg, $A^6$ is Phe, $A^7$ is Arg, $A^8$ is Pro, and $A^9$ is D-Ala; or

wherein $A^1$ is D-$\beta$-Nal, $A^2$ is D-Phe, $A^3$ is D-Phe, $A^4$ is Tyr, $A^5$ is D-Lys (isopropyl), $A^6$ is Phe, $A^7$ is Lys (isopropyl), $A^8$ is Pro, and $A^9$ is Ala-$NH_2$; or

wherein $A^1$ is D-$\beta$-Nal, $A^2$ is D-Phe, $A^3$ is D-Phe, $A^4$ is Phe, $A^5$ is D-Arg, $A^6$ is Leu, $A^7$ is Arg, $A^8$ is Pro, and $A^9$ is D-Ala; or

wherein $A^1$ is D-$\beta$-Nal, $A^2$ is D-Phe, $A^3$ is D-Phe, $A^4$ is Tyr, $A^5$ is D-Lys (isopropyl), $A^6$ is Phe, $A^7$ is Arg, $A^8$ is Pro, and $A^9$ is D-Ala-$NH_2$; or

wherein $A^1$ is D-$\beta$-Nal, $A^2$ is D-p-Cl-Phe, $A^3$ is D-Tyr, $A^4$ is Phe, $A^5$ is D-Arg, $A^6$ is Leu, $A^7$ is Arg, $A^8$ is Pro, and $A^9$ is D-Ala.

6. A pharmaceutical or veterinary composition comprising a decapeptide as claimed in any one of claims 1 to 5 and a pharmaceutically or veterinarily acceptable carrier.

7. A decapeptide as claimed in any one of claims 1 to 5 for use in human or veterinary medicine.

8. The use of a decapeptide as claimed in any one of claims 1 to 5 in the preparation of a medicament for inhibiting the release of sex hormones.

9. A method of contraception, comprising administering to a female an effective amount of a decapeptide as claimed in any one of claims 1 to 5.

10. A process for the preparation of a decapeptide as claimed in any one of claims 1 to 5, the process comprising liberating free decapeptide (or a salt) from a corresponding substrate-bound decapeptide and optionally thereafter forming a salt or the free base as appropriate.

CLAIMS FOR THE CONTRACTING STATES: AT, GR and ES

1. A process for the preparation of a decapeptide of the formula: $N\text{-}Ac\text{-}A^1\text{-}A^2\text{-}A^3\text{-}Ser\text{-}A^4\text{-}A^5\text{-}A^6\text{-}A^7\text{-}A^8\text{-}A^9$, wherein each $A^1$, $A^2$, and $A^3$, independently, is D- β -Nal, D-p-X-Phe (where X is halogen, H, $NH_2$, $NO_2$, OH, or $C_{1-3}$ alkyl), D-Trp, D-benzothienyl (2)-Ala, or D-benzothienyl (1)-Ala; $A^4$ is p-X-Phe (where X is halogen, H, $NH_2$, $NO_2$, or $C_{1-3}$ alkyl), Tyr, Lys, Arg, Leu, Trp, or Nal; $A^5$ is D-Lys, D-Tyr, D-Arg, D-Phe, β -Nal, D- β -Nal, D-Trp, D-homo-Arg, D-diethyl-homo-Arg, D-p-X-Phe (where X is halogen, H, $NH_2$, $NO_2$, or $C_{1-3}$ alkyl) or D-Lys- ε -NH-R (where R is H, a branched or straight chain $C_1$-$C_{10}$ alkyl group, or an aryl group); $A_6$ is Leu, β -Nal, p-X-Phe (where X is halogen, H, $NH_2$, $NO_2$, OH, $C_2F_5$, or $C_{1-3}$ alkyl), or Trp; $A^7$ is Arg, Lys, or Lys- ε -NH-R (where R is H, a branched or straight chain $C_1$-$C_{10}$ alkyl group, or an aryl group); $A_8$ is Pro; and $A^9$ is D-Ala, D-Ala-$NH_2$, Ala-$NH_2$, aminoisobutyric acid amide, or Gly-$NH_2$; provided that at least one of $A^2$ or $A^3$ must be D-Phe or D-Tyr, and provided further that when $A^4$ is Lys or Arg, $A^5$ must not be D-Lys, D-Arg, D-homo-Arg, D-diethyl-homo-Arg, or D-Lys- ε -NH-R, or a pharmaceutically acceptable salt thereof; the process comprising liberating free decapeptide (or a salt) from a corresponding substrate-bound decapeptide and optionally thereafter forming a salt or the free base as appropriate.

2. A process as claimed in claim 1 wherein $A^3$ is D-Phe.

3. A process as claimed in claim 1 or 2 wherein $A^2$ is D-Phe.

4. A process as claimed in claim 1, 2 or 3 wherein $A^6$ is p-X-Phe.

5. A process as claimed in claim 1 wherein $A^1$ is D-β-Nal, $A^2$ is D-p-Cl-Phe, $A^3$ is D-Phe, $A^4$ is Tyr, $A^5$ is D-Arg, $A^6$ is Phe, $A^7$ is Arg, $A^8$ is Pro, and $A^9$ is D-Ala; or

wherein $A^1$ is D-β-Nal, $A^2$ is D-Phe, $A^3$ is D-Phe, $A^4$ is Tyr, $A^5$ is D-Arg, $A^6$ is Phe, $A^7$ is Arg, $A^8$ is Pro, and $A^9$ is D-Ala; or

wherein $A^1$ is D-β-Nal, $A^2$ is D-Phe, $A^3$ is D-Phe, $A^4$ is Tyr, $A^5$ is D-Lys (isopropyl), $A^6$ is Phe, $A^7$ is Lys (isopropyl), $A^8$ is Pro, and $A^9$ is Ala-$NH_2$; or

wherein $A^1$ is D-β-Nal, $A^2$ is D-Phe, $A^3$ is D-Phe, $A^4$ is Phe, $A^5$ is D-Arg, $A^6$ is Leu, $A^7$ is Arg, $A^8$ is Pro, and $A^9$ is D-Ala; or

wherein $A^1$ is D-β-Nal, $A^2$ is D-Phe, $A^3$ is D-Phe, $A^4$ is Tyr, $A^5$ is D-Lys (isopropyl), $A^6$ is Phe, $A^7$ is Arg, $A^8$ is Pro, and $A^9$ is D-Ala-$NH_2$; or

wherein $A^1$ is D-β-Nal, $A^2$ is D-p-Cl-Phe, $A^3$ is D-Tyr, $A^4$ is Phe, $A^5$ is D-Arg, $A^6$ is Leu, $A^7$ is Arg, $A^8$ is Pro, and $A^9$ is D-Ala.

6. A process as claimed in any one of claims 1 to 5, wherein the substrate bound decapeptide is prepared by first coupling an $A^9$ residue to a substrate to form an intermediate peptide and subsequently coupling further residues to the intermediate peptide.

7. A process as claimed in any one of claims 1 to 6 wherein the substrate comprises a resin.

8. A process as claimed in claim 7, wherein the resin is a benzhydrylamine resin.